# EUROPEAN PATENT SPECIFICATION

(11) **EP 4 138 989 B1**
(45) Date of publication and mention of the grant of the patent: **01.10.2025**
(21) Application number: 20728254.2
(22) Date of filing: 23.04.2020
(51) Int. Cl.: A61N 1/36, A61N 1/05

(54) **COCHLEAR IMPLANT SYSTEM FOR TINNITUS TREATMENT**
COCHLEA-IMPLANTATSYSTEM ZUR TINNITUS-BEHANDLUNG
SYSTÈME D'IMPLANT COCHLÉAIRE POUR TRAITEMENT DES ACOUPHÈNES

(43) Date of publication of application: 01.03.2023
(73) Proprietor: Advanced Bionics AG, 8712 Staefa (CH)
(72) Inventor: DOWNING, Mark B., Valencia, California 91354 (US)
(74) Representative: Schwan Schorer & Partner mbB
(86) International application number: PCT/US2020/029530
(87) International publication number: WO 2021/216071

(56) References cited:
- WO-A1-2009/143553
- WO-A1-2012/058539
- CA-A1- 2 370 860
- US-A1- 2001 049 466
- US-B1- 6 259 951

## Description

### BACKGROUND INFORMATION

Tinnitus is often a symptom of noise-induced hearing loss but may also result from ototoxic drugs and diseases such as Meniere's, vascular conditions, ear infections, and brain tumors. Although the mechanisms of tinnitus are not well understood, treatment may include behavioral therapy, masking, and drug treatments. However, no treatment has been shown to fully address tinnitus for a majority of sufferers. Document CA-A-2 370 860 discloses a cochlear implant adapted to treat tinnitus.

The invention is defined in claims 1 and 9.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIGS. 1A and 1B illustrate exemplary cochlear implant systems.
FIG. 2 shows an exemplary configuration of the cochlear implant system of FIG. 1A.
FIG. 3 shows another exemplary configuration of the cochlear implant system of FIG. 1A.
FIG. 4 illustrates an exemplary configuration of a controller of a cochlear implant system configured to treat tinnitus.
FIG. 5 illustrates an electrode lead of a cochlear implant system configured to treat tinnitus.
FIGS. 6-7 illustrate exemplary implementations of current steering for cochlear implant systems configured to treat tinnitus.
FIG. 8 illustrates an exemplary implementation of phantom electrical stimulation for a cochlear implant system configured to treat tinnitus.
FIG. 9 illustrates exemplary gain parameters that may be employed to implement a phantom electrical stimulation configuration for a cochlear implant system configured to treat tinnitus.
FIG. 10 illustrates an exemplary method to treat tinnitus with a cochlear implant system.

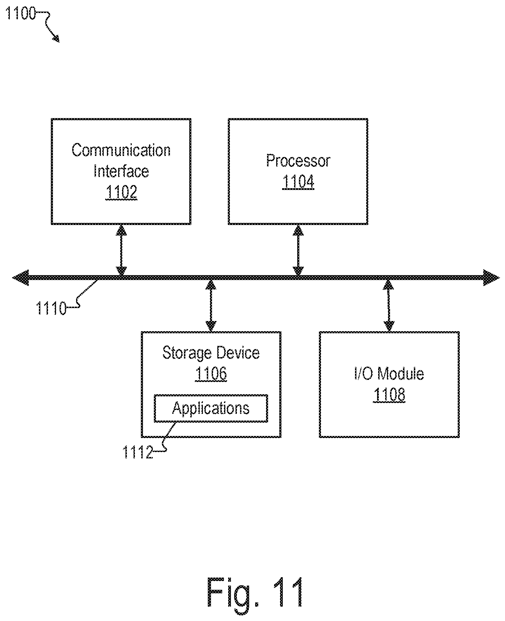

FIG. 11 illustrates an exemplary computing device.

### DETAILED DESCRIPTION

Tinnitus treatment with a cochlear implant system is described herein. To illustrate, a tinnitus system may include a cochlear implant configured to be implanted within a recipient, the cochlear implant coupled to a lead having a plurality of electrodes. The system may further include a controller communicatively coupled to the cochlear implant and configured to direct the cochlear implant to apply stimulation current to a first electrode set of the plurality of electrodes to represent the audio signal to the recipient. The controller is further configured to direct the cochlear implant to apply treatment current to a second electrode set of the plurality of electrodes to treat tinnitus within the recipient, the second electrode set including electrodes different from the first electrode set.

The systems and methods described herein may allow for treatment of tinnitus with a cochlear implant while concurrently providing electrical stimulation representing audio signals. For instance, a cochlear implant system as described herein may enable or improve hearing for a recipient of audio signals in an environment of the recipient and/or provided to the recipient via the cochlear implant system. The cochlear implant system may also provide electrical and/or acoustic stimulation to treat tinnitus in the recipient simultaneously, without having to alternate between providing stimulation representing audio signals and stimulation to treat tinnitus. These and other advantages and benefits of the present systems and methods are described in more detail herein.

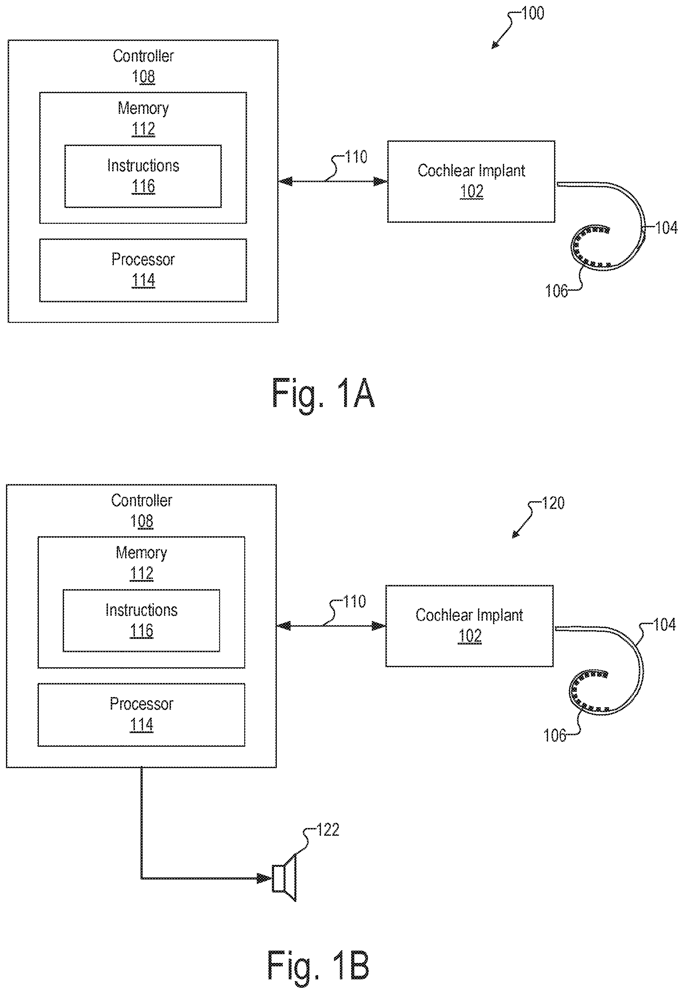

FIG. 1A illustrates an exemplary cochlear implant system 100 configured to be used by a recipient. As shown, cochlear implant system 100 includes a cochlear implant 102, an electrode lead 104 physically coupled to cochlear implant 102 and having an array of electrodes 106, and a controller 108 configured to be communicatively coupled to cochlear implant 102 by way of a communication link 110.

The cochlear implant system 100 shown in FIG. 1A is unilateral (i.e., associated with only one ear of the recipient). Alternatively, a bilateral configuration of cochlear implant system 100 may include separate cochlear implants and electrode leads for each ear of the recipient. In the bilateral configuration, controller 108 may be implemented by a single controller configured to interface with both cochlear implants or by two separate controllers each configured to interface with a different one of the cochlear implants.

Cochlear implant 102 may be implemented by any suitable type of implantable stimulator. For example, cochlear implant 102 may be implemented by an implantable cochlear stimulator. Additionally or alternatively, cochlear implant 102 may be implemented by a brainstem implant and/or any other type of device that may be implanted within the recipient and configured to apply electrical stimulation to one or more stimulation sites located along an auditory pathway of the recipient.

In some examples, cochlear implant 102 may be configured to generate electrical stimulation representative of an audio signal processed by controller 108 in accordance with one or more stimulation parameters transmitted to cochlear implant 102 by controller 108. Cochlear implant 102 may be further configured to apply the electrical stimulation to one or more stimulation sites (e.g., one or more intracochlear locations) within the recipient by way of one or more electrodes 106 on electrode lead 104. In some examples, cochlear implant 102 may include a plurality of independent current sources each associated with a channel defined by one or more of electrodes 106. In this manner, different stimulation current levels may be applied to multiple stimulation sites simultaneously by way of multiple electrodes 106.

Cochlear implant 102 may additionally or alternatively be configured to generate, store, and/or transmit data. For example, cochlear implant may use one or more electrodes 106 to record one or more signals (e.g., one or more voltages, impedances, evoked responses within the recipient, and/or other measurements) and transmit, by way of communication link 110, data representative of the one or more signals to controller 108. In some examples, this data is referred to as back telemetry data.

Electrode lead 104 may be implemented in any suitable manner. For example, a distal portion of electrode lead 104 may be pre-curved such that electrode lead 104 conforms with the helical shape of the cochlea after being implanted. Electrode lead 104 may alternatively be naturally straight or of any other suitable configuration.

In some examples, electrode lead 104 includes a plurality of wires (e.g., within an outer sheath) that conductively couple electrodes 106 to one or more current sources within cochlear implant 102. For example, if there are n electrodes 106 on electrode lead 104 and n current sources within cochlear implant 102, there may be n separate wires within electrode lead 104 that are configured to conductively connect each electrode 106 to a different one of the n current sources. Exemplary values for n are 8, 12, 16, or any other suitable number.

Electrodes 106 are located on at least a distal portion of electrode lead 104. In this configuration, after the distal portion of electrode lead 104 is inserted into the cochlea, electrical stimulation may be applied by way of one or more of electrodes 106 to one or more intracochlear locations. One or more other electrodes (e.g., including a ground electrode, not explicitly shown) may also be disposed on other parts of electrode lead 104 (e.g., on a proximal portion of electrode lead 104) to, for example, provide a current return path for stimulation current applied by electrodes 106 and to remain external to the cochlea after the distal portion of electrode lead 104 is inserted into the cochlea. Additionally or alternatively, a housing of cochlear implant 102 may serve as a ground electrode for stimulation current applied by electrodes 106.

Controller 108 may be configured to interface with (e.g., control and/or receive data from) cochlear implant 102. For example, controller 108 may transmit commands (e.g., stimulation parameters and/or other types of operating parameters in the form of data words included in a forward telemetry sequence) to cochlear implant 102 by way of communication link 110. Controller 108 may additionally or alternatively provide operating power to cochlear implant 102 by transmitting one or more power signals to cochlear implant 102 by way of communication link 110. Controller 108 may additionally or alternatively receive data from cochlear implant 102 by way of communication link 110. Communication link 110 may be implemented by any suitable number of wired and/or wireless bidirectional and/or unidirectional links.

As shown, controller 108 includes a memory 112 and a processor 114 configured to be selectively and communicatively coupled to one another. In some examples, memory 112 and processor 114 may be distributed between multiple devices and/or multiple locations as may serve a particular implementation.

Memory 112 may be implemented by any suitable non-transitory computer-readable medium and/or non-transitory processor-readable medium, such as any combination of non-volatile storage media and/or volatile storage media. Exemplary non-volatile storage media include, but are not limited to, read-only memory, flash memory, a solid-state drive, a magnetic storage device (e.g., a hard drive), ferroelectric random-access memory ("RAM"), and an optical disc. Exemplary volatile storage media include, but are not limited to, RAM (e.g., dynamic RAM).

Memory 112 may maintain (e.g., store) executable data used by processor 114 to perform one or more of the operations described herein. For example, memory 112 may store instructions 116 that may be executed by processor 114 to perform any of the operations described herein. Instructions 116 may be implemented by any suitable application, program (e.g., sound processing program), software, code, and/or other executable data instance. Memory 112 may also maintain any data received, generated, managed, used, and/or transmitted by processor 114.

Processor 114 may be configured to perform (e.g., execute instructions 116 stored in memory 112 to perform) various operations with respect to cochlear implant 102.

To illustrate, processor 114 may be configured to control an operation of cochlear implant 102. For example, processor 114 may receive an audio signal (e.g., by way of a microphone communicatively coupled to controller 108, a wireless interface (e.g., a Bluetooth interface), and/or a wired interface (e.g., an auxiliary input port)). Processor 114 may process the audio signal in accordance with a sound processing program (e.g., a sound processing program stored in memory 112) to generate appropriate stimulation parameters. Processor 114 may then transmit the stimulation parameters to cochlear implant 102 to direct cochlear implant 102 to apply electrical stimulation representative of the audio signal to the recipient.

In some implementations, processor 114 may also be configured to apply acoustic stimulation to the recipient. For example, FIG. 1B shows an exemplary configuration 120 of a cochlear implant system (e.g., cochlear implant system 100) including a receiver 122 (also referred to as a loudspeaker) coupled to controller 108. In configuration 120, processor 114 may deliver acoustic stimulation to the recipient by way of receiver 122. The acoustic stimulation may be representative of an audio signal (e.g., an amplified version of the audio signal), configured to elicit an evoked response within the recipient, and/or otherwise configured. In configuration 120 and other such configurations in which processor 114 is configured to both deliver acoustic stimulation to the recipient and direct cochlear implant 102 to apply electrical stimulation to the recipient, cochlear implant system 100 may be referred to as a bimodal hearing system and/or any other suitable term.

Processor 114 may be additionally or alternatively configured to receive and process data generated by cochlear implant 102. For example, processor 114 may receive data representative of a signal recorded by cochlear implant 102 using one or more electrodes 106 and, based on the data, adjust one or more operating parameters of controller 108. Additionally or alternatively, processor 114 may use the data to perform one or more diagnostic operations with respect to cochlear implant 102 and/or the recipient.

Other operations may be performed by processor 114 as may serve a particular implementation. In the description provided herein, any references to operations performed by controller 108 and/or any implementation thereof may be understood to be performed by processor 114 based on instructions 116 stored in memory 112.

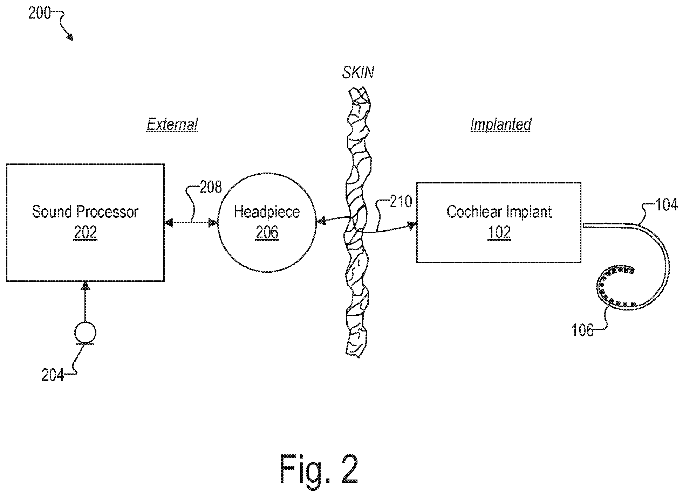

Controller 108 may be implemented by one or more devices configured to interface with cochlear implant 102. To illustrate, FIG. 2 shows an exemplary configuration 200 of cochlear implant system 100 in which controller 108 is implemented by a sound processor 202 configured to be located external to the recipient. In configuration 200, sound processor 202 is communicatively coupled to a microphone 204 and to a headpiece 206 that are both configured to be located external to the recipient.

Sound processor 202 may be implemented by any suitable device that may be worn or carried by the recipient. For example, sound processor 202 may be implemented by a behind-the-ear ("BTE") unit configured to be worn behind and/or on top of an ear of the recipient. Additionally or alternatively, sound processor 202 may be implemented by an off-the-ear unit (also referred to as a body worn device) configured to be worn or carried by the recipient away from the ear. Additionally or alternatively, at least a portion of sound processor 202 is implemented by circuitry within headpiece 206.

Microphone 204 is configured to detect one or more audio signals (e.g., that include speech and/or any other type of sound) in an environment of the recipient. Microphone 204 may be implemented in any suitable manner. For example, microphone 204 may be implemented by a microphone that is configured to be placed within the concha of the ear near the entrance to the ear canal, such as a T-MIC^{™} microphone from Advanced Bionics. Such a microphone may be held within the concha of the ear near the entrance of the ear canal during normal operation by a boom or stalk that is attached to an ear hook configured to be selectively attached to sound processor 202. Additionally or alternatively, microphone 204 may be implemented by one or more microphones in or on headpiece 206, one or more microphones in or on a housing of sound processor 202, one or more beam-forming microphones, and/or any other suitable microphone as may serve a particular implementation.

Headpiece 206 may be selectively and communicatively coupled to sound processor 202 by way of a communication link 208 (e.g., a cable or any other suitable wired or wireless communication link), which may be implemented in any suitable manner. Headpiece 206 may include an external antenna (e.g., a coil and/or one or more wireless communication components) configured to facilitate selective wireless coupling of sound processor 202 to cochlear implant 102. Headpiece 206 may additionally or alternatively be used to selectively and wirelessly couple any other external device to cochlear implant 102. To this end, headpiece 206 may be configured to be affixed to the recipient's head and positioned such that the external antenna housed within headpiece 206 is communicatively coupled to a corresponding implantable antenna (which may also be implemented by a coil and/or one or more wireless communication components) included within or otherwise connected to cochlear implant 102. In this manner, stimulation parameters and/or power signals may be wirelessly and transcutaneously transmitted between sound processor 202 and cochlear implant 102 by way of a wireless communication link 210.

In configuration 200, sound processor 202 may receive an audio signal detected by microphone 204 by receiving a signal (e.g., an electrical signal) representative of the audio signal from microphone 204. Sound processor 202 may additionally or alternatively receive the audio signal by way of any other suitable interface as described herein. Sound processor 202 may process the audio signal in any of the ways described herein and transmit, by way of headpiece 206, stimulation parameters to cochlear implant 102 to direct cochlear implant 102 to apply electrical stimulation representative of the audio signal to the recipient.

In an alternative configuration, sound processor 202 may be implanted within the recipient instead of being located external to the recipient. In this alternative configuration, which may be referred to as a fully implantable configuration of cochlear implant system 100, sound processor 202 and cochlear implant 102 may be combined into a single device or implemented as separate devices configured to communicate one with another by way of a wired and/or wireless communication link. In a fully implantable implementation of cochlear implant system 100, headpiece 206 may not be included and microphone 204 may be implemented by one or more microphones implanted within the recipient, located within an ear canal of the recipient, and/or external to the recipient.

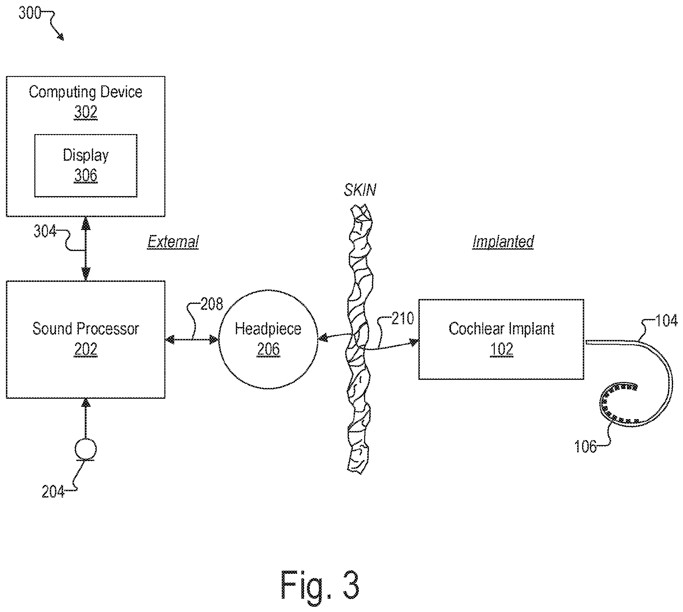

FIG. 3 shows an exemplary configuration 300 of cochlear implant system 100 in which controller 108 is implemented by a combination of sound processor 202 and a computing device 302 configured to communicatively couple to sound processor 202 by way of a communication link 304, which may be implemented by any suitable wired or wireless communication link.

Computing device 302 may be implemented by any suitable combination of hardware and software. To illustrate, computing device 302 may be implemented by a mobile device (e.g., a mobile phone, a laptop, a tablet computer, etc.), a desktop computer, and/or any other suitable computing device as may serve a particular implementation. As an example, computing device 302 may be implemented by a mobile device configured to execute an application (e.g., a "mobile app") that may be used by a user (e.g., the recipient, a clinician, and/or any other user) to control one or more settings of sound processor 202 and/or cochlear implant 102 and/or perform one or more operations (e.g., diagnostic operations) with respect to data generated by sound processor 202 and/or cochlear implant 102.

In some examples, computing device 302 may be configured to control an operation of cochlear implant 102 by transmitting one or more commands to cochlear implant 102 by way of sound processor 202. Likewise, computing device 302 may be configured to receive data generated by cochlear implant 102 by way of sound processor 202. Alternatively, computing device 302 may interface with (e.g., control and/or receive data from) cochlear implant 102 directly by way of a wireless communication link between computing device 302 and cochlear implant 102. In some implementations in which computing device 302 interfaces directly with cochlear implant 102, sound processor 202 may or may not be included in cochlear implant system 100.

Computing device 302 is shown as having an integrated display 306. Display 306 may be implemented by a display screen, for example, and may be configured to display content generated by computing device 302. Additionally or alternatively, computing device 302 may be communicatively coupled to an external display device (not shown) configured to display the content generated by computing device 302.

In some examples, computing device 302 represents a fitting device configured to be selectively used (e.g., by a clinician) to fit sound processor 202 and/or cochlear implant 102 to the recipient. In these examples, computing device 302 may be configured to execute a fitting program configured to set one or more operating parameters of sound processor 202 and/or cochlear implant 102 to values that are optimized for the recipient. As such, in these examples, computing device 302 may not be considered to be part of cochlear implant system 100. Instead, computing device 302 may be considered to be separate from cochlear implant system 100 such that computing device 302 may be selectively coupled to cochlear implant system 100 when it is desired to fit sound processor 202 and/or cochlear implant 102 to the recipient.

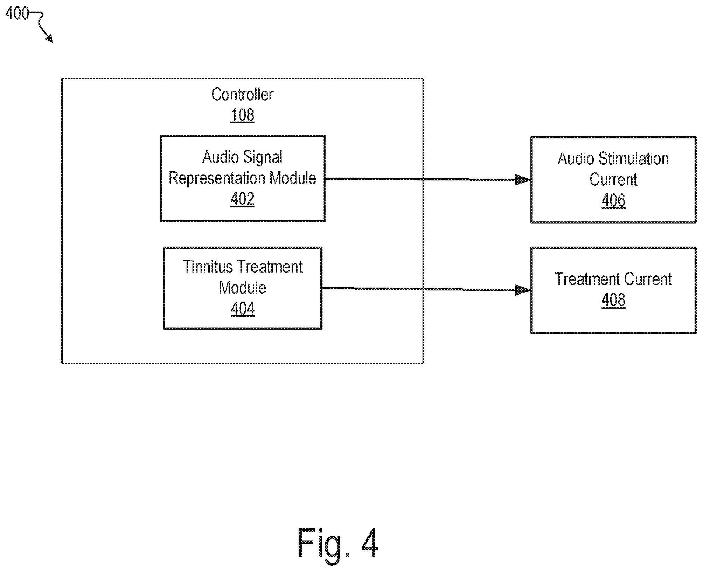

FIG. 4 illustrates an exemplary configuration 400 in which controller 108 includes an audio signal representation module 402 and a tinnitus treatment module 404.

Audio signal representation module 402 may be configured to direct cochlear implant 102 to generate electrical stimulation representative of an audio signal processed by controller 108 in any of the ways described herein. This electrical stimulation is referred to herein as audio stimulation current 406.

Tinnitus treatment module 404 may be configured to direct cochlear implant 102 to generate electrical stimulation configured to treat tinnitus in the recipient. This electrical stimulation is referred to herein as treatment current 408. Treatment current 408 may be any suitable current that is used to treat (e.g., reduce, suppress, mask, prevent, etc.) tinnitus experienced by the recipient in any suitable manner. Tinnitus treatment module 404 may control various parameters of treatment current 408 to be applied within the recipient. For instance, parameters may include frequency, pulse width, amplitude, burst pattern, stimulation site selection, etc.

As described herein, cochlear implant 102 may include a plurality of independent current sources each associated with a channel defined by one or more electrodes. In this manner, controller 108 may direct cochlear implant 102 to apply both audio stimulation current 406 and treatment current 408 concurrently. For example, controller 108 may group the electrodes into a first electrode set and a second electrode set. Controller 108 may then direct the cochlear implant to apply audio stimulation current 406 to the first electrode set and direct the cochlear implant to apply treatment current 408 to the second electrode set. Controller 108 may group the electrodes in any suitable manner.

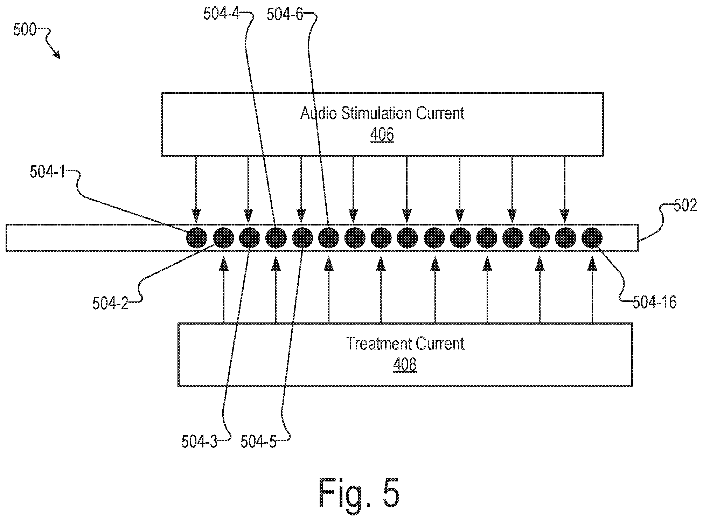

For instance, FIG. 5 illustrates a configuration 500 including an electrode lead 502 (e.g., an implementation of electrode lead 104) of a cochlear implant system (e.g., cochlear implant system 100). Electrode lead 502 includes electrodes 504 (e.g., electrode 504-1 through electrode 504-16) sequentially aligned and adjacent one to another along electrode lead 502. As shown in configuration 500, electrodes 504 are grouped into a first electrode set that includes every other electrode starting with electrode 504-1 (e.g., electrode 504-1, 504-3, 504-5, etc.). Second electrode set includes every other electrode starting with electrode 504-2 (e.g., electrode 504-2, 504-4, 504-6, etc.). Audio stimulation current 406 is applied by the first electrode set and treatment current 408 is applied by the second electrode set. Alternatively, audio stimulation current 406 may be applied by the second electrode set and treatment current 408 applied by the first electrode set. In this manner, audio stimulation current 406 and treatment current 408 may be applied concurrently, allowing the cochlear implant system to provide audio signals to the recipient while simultaneously treating tinnitus in the recipient.

While configuration 500 shows one particular grouping of electrodes into the first electrode set and the second electrode set, any suitable grouping may be used. For example, the grouping may be based on stimulation sites corresponding to perceived frequencies associated with each electrode. For instance, electrodes 504 may each be associated with a respective stimulation site that has a respective particular perceived frequency (e.g., place pitch, as described herein). The second (or first) electrode set may include electrodes associated with stimulation sites corresponding to perceived frequencies that are below a threshold frequency (e.g., a maximum frequency used for treatment current 408), while the first (or second) electrode set may include electrodes associated with stimulation sites corresponding to perceived frequencies that include and are above the threshold frequency. For example, some tinnitus treatment schemes may involve stimulation below threshold frequencies (e.g., 1500 hertz (Hz), or any other suitable threshold). For such schemes, the second set of electrodes may include electrodes associated with stimulation sites corresponding to perceived frequencies below the threshold. Alternatively, the second set of electrodes may include a subset of the electrodes associated with stimulation sites corresponding to perceived frequencies below the threshold (e.g., every other, every third, or any other suitable subset of the electrodes).

Additionally or alternatively, the grouping of the first and second electrode sets may dynamically change based on audio stimulation current 406 and/or treatment current 408. For example, the cochlear implant system may access (e.g., receive, generate, determine, etc.) a tinnitus treatment regimen, which may include one or more stimulation currents applied for one or more specific periods of time. Based on such a regimen, a change in the regimen, and/or a change to a different regimen, the cochlear implant system may dynamically change the first electrode set and/or the second electrode set to include different electrodes of electrodes 504. For instance, electrodes 504 may all be included in the first electrode set to provide audio stimulation current 406 outside the one or more specific periods of time, while all or some of electrodes 504 are included in the second electrode set to provide treatment current 408 during the one or more specific periods of time. Additionally or alternatively, electrodes 504 may all be included in the second electrode set to provide treatment current 408 until an audio signal is received by controller 108 to provide to the recipient, at which point some or all of electrodes 504 may be included in the first electrode set to provide audio stimulation current 406. Example regimens may include time periods for treatment throughout a course of a day (e.g., a treatment in the morning, several treatments throughout the day, one or more treatments at night, etc.). Such time periods and/or treatment regimens may be initiated by a user of the cochlear implant system and/or initiated by the cochlear implant system based on a program parameter indicating treatment time periods and/or regimens.

Controller 108 is configured to direct cochlear implant 102 to use current steering to provide audio stimulation current 406 to stimulation sites located in between stimulation sites associated with the electrodes included in the first electrode set. In configuration 500, audio stimulation current 406 includes weighted amounts of stimulation current applied to specific electrodes of the first electrode set to stimulate one or more stimulation sites located in between the specific electrodes. The locations are stimulation sites associated with electrodes in the second electrode set. As an example, audio stimulation current 406 may include weighted amounts of stimulation current provided to electrode 504-1 and electrode 504-3 to stimulate a stimulation site in between stimulation sites associated with electrode 504-1 and electrode 504-3 (e, g. a stimulation site associated with electrode 504-2, a stimulation site in between electrode 504-1 and electrode 504-2, a stimulation site in between electrode 504-2, electrode 504-3, etc.). In this manner, by using current steering, audio stimulation current 406 may provide stimulation using the first electrode set that may be substantially similar to stimulation that may be provided using all of electrodes 504 or more of electrodes 504 than the first electrode set. Thus, the cochlear implant system may provide a full or substantially full range of audio signal representation while concurrently providing tinnitus treatment. Similarly, in some examples, treatment current 408 may also use current steering to provide stimulation to stimulation sites between stimulation sites associated with electrodes of the second electrode set. Example implementations of current steering are further described herein.

Additionally, controller 108 may direct cochlear implant 102 to apply phantom electrical stimulation to the first and second electrode sets to stimulate stimulation sites associated with an apical or a basal portion of a cochlea of the recipient. For example, treatment current 408 and/or audio stimulation current 406 may include an amount of current to a most apical electrode (or a most basal electrode) of an electrode set along with one or more compensating electrodes of the electrode set to stimulate stimulation sites located more apically (or more basally) than the most apical electrode (or most basal) electrode. Example implementations of phantom electrical stimulation are further described herein.

Additionally or alternatively, a tinnitus treatment regimen may include compressed analog stimulation, simultaneous analog stimulation, pulsatile stimulation, acoustic stimulation, and/or any combination of such stimulation schemes. Any such suitable tinnitus treatment regimen may be applied by or in conjunction with treatment current 408. For instance, controller 108 may direct a receiver of the cochlear implant system to apply acoustic stimulation in conjunction with treatment current 408 to treat tinnitus within the recipient. Such acoustic stimulation may be targeted at any suitable location, such as a vagus nerve of the recipient.

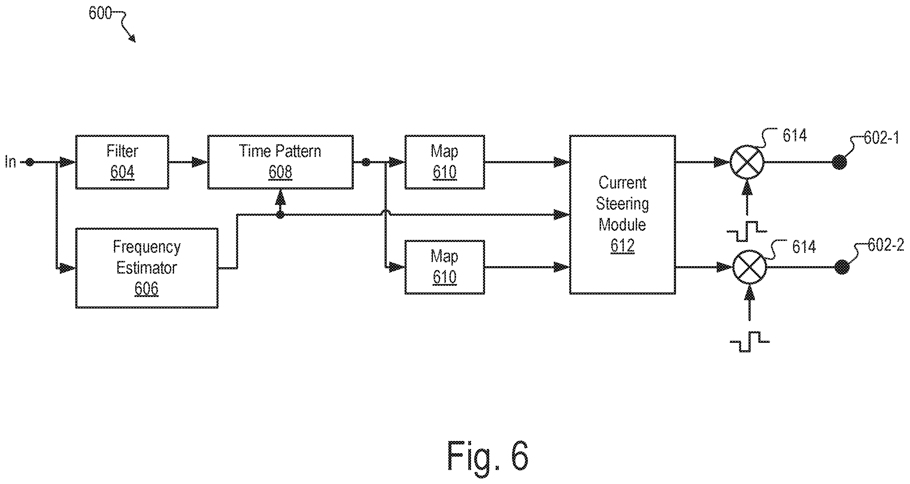

FIG. 6 illustrates an exemplary implementation 600 of current steering that may be used in connection with any of the stimulation strategies described herein. The components and functions illustrated in FIG. 6 may be implemented by any of the systems, facilities, and/or modules described herein. For example, one or more components of controller 108 may be configured to perform any of the functions described in connection with FIG. 6.

As shown in FIG. 6, current steering may be applied to two or more electrodes 602 (e.g., electrodes 602-1 and 602-2). Two electrodes 602 are shown in FIG. 6 for illustrative purposes only. It will be recognized that current steering may alternatively be applied to three or more electrodes as may serve a particular application. Electrodes 602-1 and 602-2 may be adjacent one to another (i.e., no other electrode 602 is physically disposed in between them on a lead). Alternatively, electrodes 602-1 and 602-2 may be non-adjacent (i.e., one or more electrodes 602 are physically disposed in between them on a lead).

As shown in FIG. 6, an input signal may be filtered by at least one filter 604 configured to generate a frequency domain signal representative of a distinct frequency portion of the audio signal. The input signal is also input into a frequency estimator 606 configured to estimate the peak frequency thereof. A time pattern block 608 is configured to build the temporal structure of a pulse train representing the signal output by the at least one filter 604. Mapping modules 610 are configured to map the amplitude of the signal output by time pattern block 608 to corresponding current levels in accordance with a suitable mapping function.

The output of each mapping module 610 is input into a current steering module 612. Current steering module 612 is also configured to receive the output of frequency estimator 606. In some examples, current steering module 612 is configured to determine appropriate weighting factors for current to be applied to electrodes 602-1 and 602-2. This determination may be based at least in part on the peak frequency estimate and the output of each of mapping modules 610. The weighting factors may be applied to the current using multiplication blocks 614. In this manner, stimulation current may be delivered to a stimulation site located in between areas associated with electrodes 602-1 and 602-2.

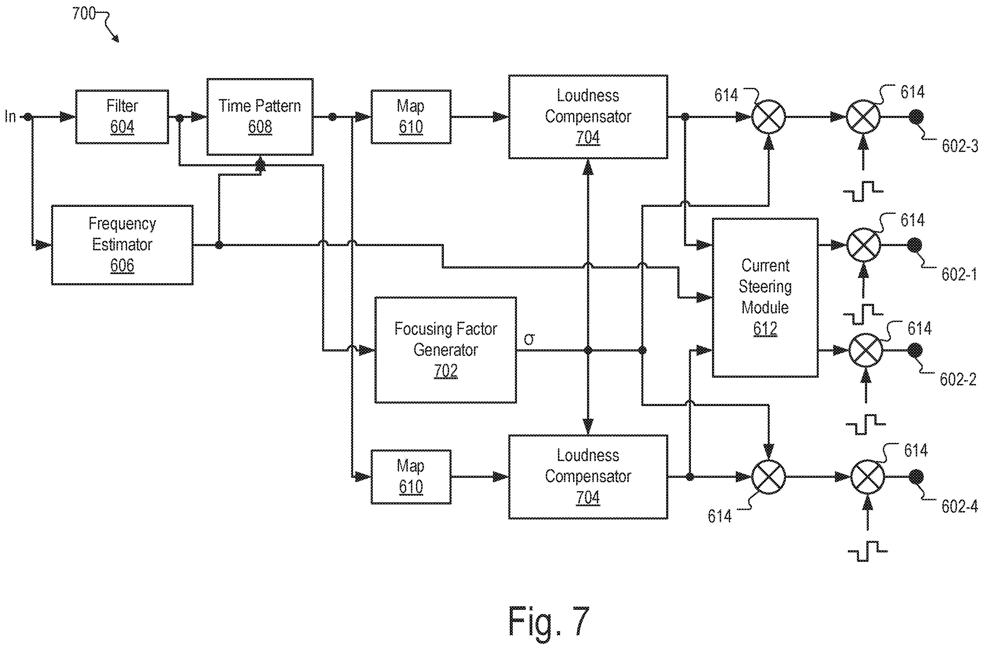

The excitation field produced by the current steering electrodes 602-1 and 602-2 may be focused by applying compensating current simultaneously to one or more additional electrodes (referred to herein as compensating electrodes). To illustrate, FIG. 7 illustrates another exemplary implementation 700 of a current steering strategy that may be used to dynamically focus or defocus one or more excitation fields produced by current steering electrodes (e.g., electrodes 702-1 and 702-2). The components and functions illustrated in FIG. 7 may be implemented by any of the systems, facilities, and/or modules described herein. For example, one or more components of controller 108 may be configured to perform any of the functions described in connection with FIG. 7.

Implementation 700 includes many of the same components as the implementation described in connection with FIG. 6. In addition, implementation 700 includes a focusing factor generator 702 configured to generate focusing factor σ based on the amplitude of the signal output by filter 604. The focusing factor σ is used to generate scaled versions of the current steering current. This scaled current is delivered via one or more additional electrodes (e.g., electrodes 602-3 and 602-4) to effectively focus or defocus the excitation field produced by electrodes 602-1 and 602-2.

As shown in FIG. 7, loudness compensators 704 may also be included within implementation 700. Loudness compensators 704 are configured to adjust the amplitudes of the currents applied via electrodes 602-1 and 602-2 to compensate for loudness changes that may be caused by current delivered via the compensating electrodes 602-3 and 602-4.

While exemplary implementations 600 and 700 of current steering have been described herein, it will be recognized that other implementations of current steering may be additionally or alternatively used in connection with the systems and methods described herein as may serve a particular implementation. Current steering is described in more detail in U.S. Patent No. 9,597,502.

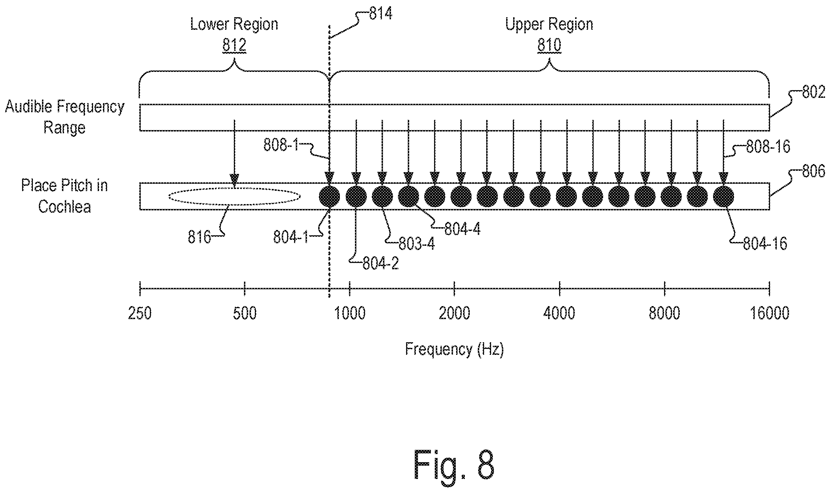

FIG. 8 illustrates an exemplary implementation of phantom electrical stimulation. FIG. 8 shows a mapping between frequencies in an audible frequency range 802 and electrodes 804 (e.g., electrodes 804-1 through 804-16) that may be defined by a frequency allocation table. As described herein, the frequency allocation table may be used by controller 108 to direct cochlear implant 102 to apply electrical stimulation representative of various frequencies included in an audio signal.

For purposes of this example, audible frequency range 802 includes a range of frequencies including and in between 250 Hz and 16 kHz. Each of these frequencies may be audible to a person with normal hearing. In some examples, the frequencies in audible frequency range 802 are also audible to a hearing impaired recipient of a cochlear implant system. It will be recognized that some frequencies lower than 250 Hz and some frequencies above 16 kHz may be included in audible frequency range 802, depending on the particular person and/or listening scenario as may serve a particular implementation.

In the example of FIG. 8, electrode 804-1 is the most apical electrode on an electrode lead 806 (e.g., electrode lead 104). In other words, electrode 804-1 is the most distally located electrode on electrode lead 806 such that when the electrode lead is inserted into the cochlea, electrode 804-1 is located closest to the apex of the cochlea. Electrode 804-16 is the most basal electrode on the electrode lead. In other words, electrode 804-16 is the most proximally located electrode on the electrode lead such that when the electrode lead is inserted into the cochlea, electrode 804-16 is located closest to the base of the cochlea.

Once implanted within the cochlea, electrodes 804 may each be located at a different intracochlear location that corresponds to a particular place pitch 806. As used herein, a "place pitch" associated with a particular intracochlear location refers to a frequency that is perceived by the recipient when the intracochlear location is stimulated with electrical stimulation by an electrode 804 at the intracochlear location. For example, as shown, electrode 804-1 is located at an intracochlear location (e.g., a stimulation site) associated with a place pitch of approximately 700 Hz and electrode 804-16 is located at an intracochlear location associated with a place pitch of approximately 14 kHz.

Arrows (e.g., arrow 808-1 through arrow 808-16) represent mappings defined by a frequency allocation table between various frequencies in audible frequency range 802 and electrodes 804. As shown, audible frequency range 802 may be divided into an upper region 810 and a lower region 812. Upper region 810 includes a cutoff frequency 814 and frequencies above cutoff frequency 814. Lower region 812 includes frequencies below cutoff frequency 814. In the example of FIG. 8, the cutoff frequency 814 is approximately 700 Hz. Hence, upper region 810 includes frequencies between and including 700 Hz and 16 kHz, and lower region 812 includes frequencies below 700 Hz.

In this configuration, based on the mapping illustrated in FIG. 8, standard electrical stimulation is not used to convey frequencies in lower region 810. Rather, the controller is configured to direct the cochlear implant system to convey frequencies in lower region 810 by applying phantom electrical stimulation by way of a phantom stimulation channel 816 in accordance with a phantom electrode stimulation configuration. Phantom stimulation channel 816 comprises the most apical electrode 804-1 and one or more compensating electrodes 804. In some examples, compensating electrodes 804 are adjacent to most apical electrode 804-1 (e.g., electrode 804-2 and/or electrode 804-3).

As described herein, phantom electrical stimulation is configured to convey (e.g., cause a recipient to perceive) a frequency that is not mapped to an electrode in a frequency allocation table (e.g., in a frequency allocation table that has the mapping illustrated in FIG. 8). For example, phantom electrical stimulation applied by way of most apical electrode 804-1 and one or more compensating electrodes adjacent to the most apical electrode may convey a frequency or pitch that is lower than the frequency mapped to most apical electrode 804-1 in the frequency allocation table.

Controller 108 may be configured to direct cochlear implant 102 to apply a main stimulation current by way of the most apical electrode 804-1, directing the cochlear implant to concurrently apply, while the main stimulation current is being applied by way of the most apical electrode 804-1, a compensation stimulation current by way of the one or more compensating electrodes (e.g., electrodes 804-2 and/or 804-3), and optimizing an amount of the compensation stimulation current to result in the frequencies in the audio signal that are within lower region 812 of audible frequency range 802 being presented to the recipient.

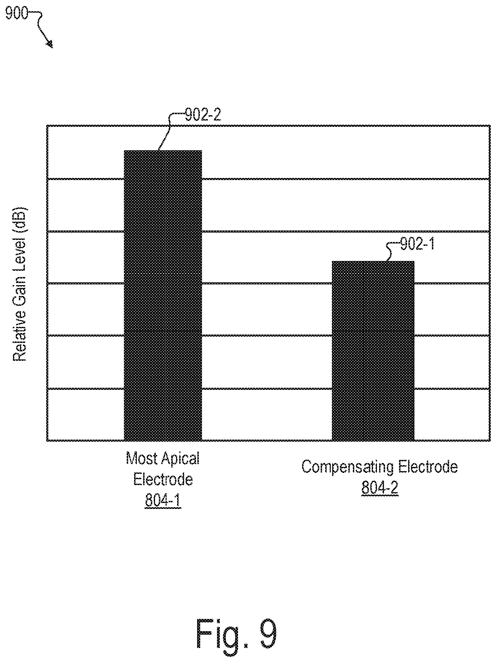

FIG. 9 illustrates exemplary gain parameters that may be employed to implement a phantom electrical stimulation configuration. In this example, electrode 804-2 is the sole compensating electrode. As depicted in FIG. 8, the controller may adjust relative gain levels of gain parameters 902-1 and 902-2 corresponding to compensating electrode 804-2 and most apical electrode 804-1, respectively, such that a frequency perceived by a recipient is substantially identical to a frequency in an audio signal that is within lower region 812. Gain parameter 902-1 may represent a level of compensation stimulation current applied by way of compensating electrode 804-1 and gain parameter 902-2 may represent a level of main stimulation current applied by way of most apical electrode 804-1.

In some examples, gain parameters 902-1 and 902-2 may be configured in accordance with a selected ratio of compensation stimulation current to main stimulation current corresponding to the particular frequency in the incoming audio signal. Additionally, gain parameters 902-1 and 902-2 may be adjusted such that the total current applied to electrodes 804-1 and 804-2 is substantially at the most comfortable current level. In some examples, the compensation stimulation current is out-of-phase with main current (e.g., by 180 degrees). The compensation stimulation current may additionally or alternatively have a polarity opposite that of the main stimulation current.

Frequencies in lower region 812 of audible frequency range 802 may additionally be conveyed to a recipient in any other suitable manner. For example, in cases where a recipient is associated with a bimodal hearing system (e.g., configuration 120), frequencies in lower region 812 of audible frequency range 802 may additionally be conveyed by way of a receiver (e.g., receiver 122) of the bimodal hearing system.

Phantom electrode stimulation is described in more detail in U.S. Patent No. 9,056,205.

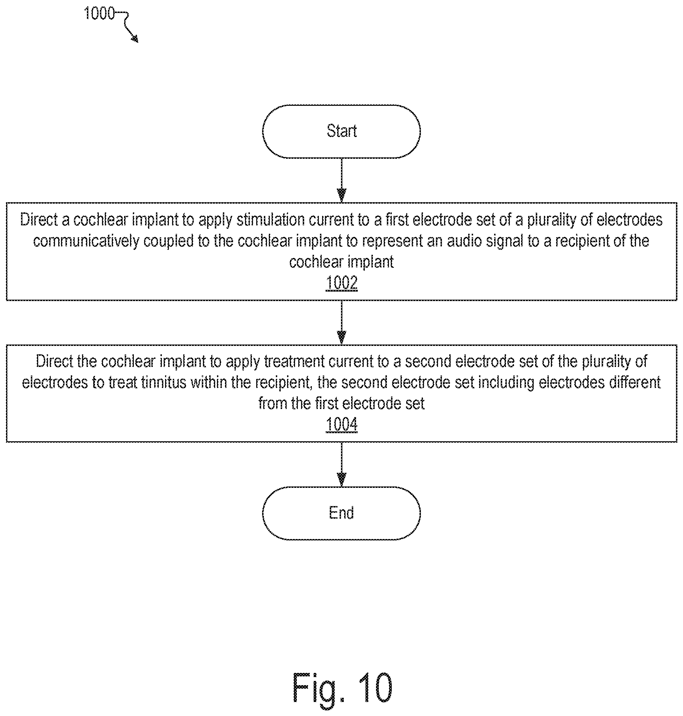

FIG. 10 illustrates an exemplary method 1000. The operations shown in FIG. 10 may be performed by controller 108 and/or any implementation thereof. While FIG. 10 illustrates exemplary operations according to one embodiment, other embodiments may omit, add to, reorder, and/or modify any of the operations shown in FIG. 10.

In operation 1002, a controller directs a cochlear implant to apply stimulation current to a first electrode set of a plurality of electrodes communicatively coupled to the cochlear implant to represent an audio signal to a recipient of the cochlear implant. Operation 1002 may be performed in any of the ways described herein.

In operation 1004, the controller directs the cochlear implant to apply treatment current to a second electrode set of the plurality of electrodes to treat tinnitus within the recipient, the second electrode set including electrodes different from the first electrode set. Operation 1004 may be performed in any of the ways described herein.

In the preceding description, various exemplary embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense.

## Claims

1. A system comprising:
a cochlear implant (102) configured to be implanted within a recipient, the cochlear implant coupled to a lead (104) having a plurality of electrodes;
a controller (108) communicatively coupled to the cochlear implant and configured to:
direct the cochlear implant to apply stimulation current to a first electrode set of the plurality of electrodes to represent the audio signal to the recipient; and
direct the cochlear implant to apply treatment current to a second electrode set of the plurality of electrodes to treat tinnitus within the recipient, the second electrode set including electrodes different from the first electrode set, **characterized in that**
the directing of the cochlear implant to apply the stimulation current comprises directing the cochlear implant to apply weighted amounts of the stimulation current to a pair of electrodes of the first electrode set in accordance with a current steering strategy to stimulate a stimulation site located in between stimulation sites associated with the pair of electrodes,
wherein: the stimulation site is associated with an electrode of the second electrode set.

2. The system of claim 1, wherein the applying of the stimulation current to the first electrode set is concurrent to the applying of the treatment current to the second electrode set.

3. The system of claim 1, wherein the controller is further configured to:
access a treatment regimen to treat tinnitus in the recipient; and
based on the treatment regimen or a change in the treatment regimen, dynamically change at least one of the first electrode set and the second electrode set to include a different set of the plurality of electrodes.

4. The system of claim 1, wherein:
the plurality of electrodes comprises a plurality of sequentially arranged electrodes including a first electrode, a second electrode adjacent to the first electrode, a third electrode adjacent to the second electrode, and a fourth electrode adjacent to the third electrode;
the first electrode set includes the first and third electrodes; and
the second electrode set includes the second and fourth electrodes.

5. The system of claim 1, wherein the second electrode set consists of electrodes of the plurality of electrodes associated with stimulation sites corresponding to perceived frequencies that are below a threshold frequency.

6. The system of claim 5, wherein the threshold frequency is 1500 hertz (Hz).

7. The system of claim 1, wherein the applying the treatment current includes applying phantom electrical stimulation by way of a most apical electrode or a most basal electrode of the second electrode set and one or more compensating electrodes of the second electrode set.

8. The system of claim 1, further comprising a receiver coupled to the controller, and wherein the controller is further configured to direct the receiver to apply acoustic stimulation to the recipient in conjunction with the applying the treatment current to treat tinnitus within the recipient.

9. A system comprising:
a memory storing instructions;
a controller communicatively coupled to the memory and configured to execute the instructions to:
direct a cochlear implant to apply stimulation current to a first electrode set of a plurality of electrodes communicatively coupled to the cochlear implant to represent an audio signal to a recipient of the cochlear implant; and
direct the cochlear implant to apply treatment current to a second electrode set of the plurality of electrodes to treat tinnitus within the recipient, the second electrode set including electrodes different from the first electrode set, **characterized in that** the directing of the cochlear implant to apply the stimulation current comprises directing the cochlear implant to apply weighted amounts of the stimulation current to a pair of electrodes of the first electrode set in accordance with a current steering strategy to stimulate a stimulation site located in between stimulation sites associated with the pair of electrodes,
wherein:
the stimulation site is associated with an electrode of the second electrode set.

10. The system of claim 9, wherein the applying of the stimulation current to the first electrode set is concurrent to the applying of the treatment current to the second electrode set.

11. The system of claim 10, wherein:
the plurality of electrodes comprises a plurality of sequentially arranged electrodes including a first electrode, a second electrode adjacent to the first electrode, a third electrode adjacent to the second electrode, and a fourth electrode adjacent to the third electrode;
the first electrode set includes the first and third electrodes; and
the second electrode set includes the second and fourth electrodes.

## Patentansprüche

1. System mit:
einem Cochlea-Implantat (102) zum Implantieren innerhalb eines Empfängers, welches mit einer Zuleitung (104) mit einer Mehrzahl von Elektroden gekoppelt ist;
einem Steuergerät (108), welches mit dem Cochlea-Implantat kommunikativ gekoppelt ist und ausgebildet ist, um:
das Cochlea-Implantat zu veranlassen, einen Stimulationsstrom auf einen ersten Elektrodensatz der Mehrzahl von Elektroden anzuwenden, um das Audiosignal dem Empfänger darzubieten; und
das Cochlea-Implantat zu veranlassen, einen Behandlungsstrom auf einen zweiten Elektrodensatz der Mehrzahl von Elektroden anzuwenden, um Tinnitus innerhalb des Empfängers zu behandeln, wobei der zweite Elektrodensatz Elektroden beinhaltet, die sich von dem ersten Elektrodensatz unterscheiden,
**dadurch gekennzeichnet, dass**
beim Veranlassen des Cochlea-Implantats zum Anwenden des Stimulationsstroms das Cochlea-Implantat veranlasst wird, gewichtete Beträge des Stimulationsstroms auf ein Elektrodenpaar des ersten Elektrodensatzes gemäß einer Stromsteuerstrategie anzuwenden, um eine Stimulationsstelle zu stimulieren, welche zwischen mit dem Elektrodenpaar assoziierten Stimulationsstellen liegt,
wobei die Stimulationsstelle mit einer Elektrode des zweiten Elektrodensatzes assoziiert ist.

2. System gemäß Anspruch 1, wobei das Anwenden des Stimulationsstroms auf den ersten Elektrodensatz gleichzeitig mit dem Anwenden des Behandlungsstroms auf den zweiten Elektrodensatz erfolgt.

3. System gemäß Anspruch 1, wobei das Steuergerät ferner ausgebildet ist, um:
auf ein Behandlungsregime zuzugreifen, um Tinnitus in dem Empfänger zu behandeln;
basierend auf dem Behandlungsregime oder einer Veränderung des Behandlungsregimes den ersten Elektrodensatz und/oder den zweiten Elektrodensatz dahingehend dynamisch zu verändern, dass ein unterschiedlicher Satz der Mehrzahl von Elektroden beinhaltet ist.

4. System gemäß Anspruch 1, wobei die Mehrzahl von Elektroden eine Mehrzahl von sequentiell angeordneten Elektroden einschließlich einer ersten Elektrode, einer zweiten Elektrode benachbart zu der ersten Elektrode, einer dritten Elektrode benachbart zu der zweiten Elektrode sowie einer vierten Elektrode benachbart zu der dritten Elektrode aufweist;
wobei der erste Elektrodensatz die erste und die dritte Elektrode beinhaltet; und
wobei der zweite Elektrodensatz die zweite und die dritte Elektrode beinhaltet.

5. System gemäß Anspruch 1, wobei der zweite Elektrodensatz aus Elektroden der Mehrzahl von Elektroden besteht, die mit Stimulationsstellen assoziiert sind, die wahrgenommenen Frequenzen entsprechen, die unterhalb einer Schwellwertfrequenz liegen.

6. System gemäß Anspruch 5, wobei die Schwellwertfrequenz 1500 Hertz (Hz) beträgt.

7. System gemäß Anspruch 1, wobei das Anwenden des Behandlungsstroms beinhaltet, dass elektrische Phantomstimulation mittels einer am meisten apikal gelegenen Elektrode oder einer am meisten basal gelegenen Elektrode des zweiten Elektrodensatzes sowie mindestens einer Kompensationselektrode des zweiten Elektrodensatzes angewandt wird.

8. System gemäß Anspruch 1, ferner versehen mit einem mit dem Steuergerät gekoppelten Lautsprecher, wobei das Steuergerät ferner ausgebildet ist, um den Lautsprecher zu veranlassen, akustische Stimulation auf den Empfänger in Verbindung mit dem Anwenden des Behandlungsstrom zum Behandeln von Tinnitus innerhalb des Empfängers anzuwenden.

9. System mit:
einem Speicher zum Speichern von Anweisungen;
einem mit dem Speicher kommunikativ gekoppelten Steuergerät zum Ausführen der Anweisungen, um:
ein Cochlea-Implantat zu veranlassen, Stimulationsstrom auf einen ersten Elektrodensatz einer Mehrzahl von mit dem Cochlea-Implantat kommunikativ gekoppelten Elektroden anzuwenden, um einem Empfänger des Cochlea-Implantats ein Audiosignal darzubieten; und
das Cochlea-Implantat zu veranlassen, Behandlungsstrom auf einen zweiten Elektrodensatz der Mehrzahl von Elektroden anzuwenden, um Tinnitus innerhalb des Empfängers zu behandeln, wobei der zweite Elektrodensatz Elektroden beinhaltet, die sich von dem ersten Elektrodensatz unterscheiden,
**dadurch gekennzeichnet, dass**
beim Veranlassen des Cochlea-Implantats zum Anwenden des Stimulationsstroms das Cochlea-Implantat veranlasst wird, gewichtete Beträge des Stimulationsstroms auf ein Elektrodenpaar des ersten Elektrodensatzes gemäß einer Stromsteuerstrategie anzuwenden, um eine Stimullatiionsstelle zu stimulieren, die zwiischen mit dem Elektrodenpaar assoziierten Stimulationsstellen liegt,
wobei die Stimulationsstelle mit einer Elektrode des zweiten Elektrodensatzes assoziiert ist.

10. System gemäß Anspruch 9, wobei das Anwenden des Stimulationsstroms auf den ersten Elektrodensatz gleichzeitig mit dem Anwenden des Behandlungsstrom auf den zweiten Elektrodensatz erfolgt.

11. System gemäß Anspruch 10, wobei:
die Mehrzahl von Elektroden eine Mehrzahl von sequentiell angeordneten Elektroden mit einer ersten Elektrode, einer zweiten Elektrode benachbart zu der ersten Elektrode, einer dritten Elektrode benachbart zu der zweiten Elektrode sowie einer vierten Elektrode benachbart zu der dritten Elektrode beinhaltet;
der erste Elektrodensatz die erste und die dritte Elektrode beinhaltet; und
der zweite Elektrodensatz die zweite und die vierte Elektrode beinhaltet.

## Revendications

1. Système comprenant :
un implant cochléaire (102) conçu pour être implanté dans le corps d'un receveur, l'implant cochléaire étant couplé à un fil (104) comportant une pluralité d'électrodes ;
un contrôleur (108) couplé de façon communicante à l'implant cochléaire et conçu pour :
donner l'instruction à l'implant cochléaire d'appliquer un courant de stimulation à un premier jeu d'électrodes de la pluralité d'électrodes pour représenter le signal audio pour le receveur ; et
donner l'instruction à l'implant cochléaire d'appliquer un courant de traitement à un deuxième jeu d'électrodes de la pluralité d'électrodes pour traiter des acouphènes chez le receveur, le deuxième jeu d'électrodes comportant des électrodes différentes de celles du premier jeu d'électrodes,
**caractérisé en ce que**
l'instruction à l'implant cochléaire d'appliquer le courant de stimulation comprend l'instruction à l'implant cochléaire d'appliquer des quantités pondérées du courant de stimulation à une paire d'électrodes du premier jeu d'électrodes selon une stratégie de guidage du courant pour stimuler, un site de stimulation situé entre des sites de stimulation associés à la paire d'électrodes,
dans lequel :
le site de stimulation est associé à une électrode du deuxième jeu d'électrodes.

2. Système selon la revendication 1, dans lequel l'application du courant de stimulation au premier jeu d'électrodes est concomitante à l'application du courant de traitement au deuxième jeu d'électrodes.

3. Système selon la revendication 1, dans lequel le contrôleur est en outre conçu pour :
accéder à un schéma thérapeutique pour traiter des acouphènes chez le receveur ; et
sur la base du schéma thérapeutique ou d'un changement dans le schéma thérapeutique, changer dynamiquement le premier jeu d'électrodes et/ou le deuxième jeu d'électrodes pour inclure un jeu différent de la pluralité d'électrodes.

4. Système selon la revendication 1, dans lequel:
la pluralité d'électrodes comprend une pluralité d'électrodes disposées successivement incluant une première électrode, une deuxième électrode adjacente à la première électrode, une troisième électrode adjacente à la deuxième électrode, et une quatrième électrode adjacente à la troisième électrode ;
le premier jeu d'électrodes comporte la première et la troisième électrode ; et
le deuxième jeu d'électrodes comporte la deuxième et la quatrième électrode.

5. Système selon la revendication 1, dans lequel le deuxième jeu d'électrodes consiste en des électrodes de la pluralité d'électrodes associées à des sites de stimulation correspondant à des fréquences perçues qui sont inférieures à une fréquence seuil.

6. Système selon la revendication 5, dans lequel la fréquence seuil est égale à 1500 hertz (Hz).

7. Système selon la revendication 1, dans lequel l'application du courant de traitement comporte l'application d'une stimulation électrique fantôme par le biais d'une électrode la plus apicale ou d'une électrode la plus basale du deuxième jeu d'électrodes et d'une ou plusieurs électrodes de compensation du deuxième jeu d'électrodes.

8. Système selon la revendication 1, comprenant en outre un récepteur couplé au contrôleur, et dans lequel le contrôleur est en outre conçu pour donner l'instruction au récepteur d'appliquer une stimulation acoustique au receveur conjointement avec l'application du courant de traitement pour traiter des acouphènes chez le receveur.

9. Système comprenant :
une mémoire stockant des instructions ;
un contrôleur couplé de façon communicante à la mémoire et conçu pour exécuter les instructions afin de :
donner l'instruction à un implant cochléaire d'appliquer un courant de stimulation à un premier jeu d'électrodes d'une pluralité d'électrodes couplées de façon communicante à l'implant cochléaire pour représenter un signal audio pour un receveur de l'implant cochléaire ; et
donner l'instruction à l'implant cochléaire d'appliquer un courant de traitement à un deuxième jeu d'électrodes de la pluralité d'électrodes pour traiter des acouphènes chez le receveur, le deuxième jeu d'électrodes comportant des électrodes différentes de celles du premier jeu d'électrodes,
**caractérisé en ce que**
l'instruction à l'implant cochléaire d'appliquer le courant de stimulation comprend l'instruction à l'implant cochléaire d'appliquer des quantités pondérées du courant de stimulation à une paire d'électrodes du premier jeu d'électrodes selon une stratégie de guidage du courant pour stimuler un site de stimulation situé entre des sites de stimulation associés à la paire d'électrodes, dans lequel :
le site de stimulation est associé à une électrode du deuxième jeu d'électrodes.

10. Système selon la revendication 9, dans lequel l'application du courant de stimulation au premier jeu d'électrodes est concomitante à l'application du courant de traitement au deuxième jeu d'électrodes.

11. Système selon la revendication 10,
la pluralité d'électrodes comprend une pluralité d'électrodes disposées successivement incluant une première électrode, une deuxième électrode adjacente à la première électrode, une troisième électrode adjacente à la deuxième électrode, et une quatrième électrode adjacente à la troisième électrode ;
le premier jeu d'électrodes comporte la première et la troisième électrode ; et
le deuxième jeu d'électrodes comporte la deuxième et la quatrième électrode.
